# EUROPEAN PATENT SPECIFICATION

(11) **EP 1 601 409 B1**
(45) Date of publication and mention of the grant of the patent: **28.04.2010**
(21) Application number: 04708811.7
(22) Date of filing: 06.02.2004
(51) Int. Cl.: A61M 35/00, B05B 15/12

(54) **TANNING BOOTH**
BRÄUNUNGSKABINE
CABINE DE BRONZAGE

(30) Priority: 07.02.2003 GB 0302853; 21.03.2003 GB 0306553; 10.10.2003 GB 0323794
(43) Date of publication of application: 07.12.2005
(73) Proprietor: Beauty Source Ltd., Chilwell, Nottingham NG9 6QG (GB)
(72) Inventor: SMITH, Warrick J., Worthing, West Sussex BN14 7PS (GB); STAFFORD-NELSON, Christopher, Coventry CV3 6BY (GB)
(74) Representative: Johnstone, Helen Margaret
(86) International application number: PCT/GB2004/000472
(87) International publication number: WO 2004/069321

(56) References cited:
- EP-A- 1 238 642
- WO-A-00/54892
- WO-A-00/62640
- US-A- 5 664 593
- US-B1- 6 264 547

## Description

This invention relates to a booth in which a person may have a product such as a cosmetic product applied to her skin. The invention relates particularly to a tanning booth in which a person positioned within the booth may have a sunless tanning lotion applied to her skin.

Recently public awareness of the harmful side effects of the sun's rays has been raised. It is well known that too much exposure to the sun can lead to skin cancer. Because of this risk, the popularity of cosmetic products which provide a "sunless" tan has increased significantly over recent years.

Existing sunless tanning lotions are often in the form of creams or lotions that may be self applied to the skin. A problem with such creams and lotions is that it is very difficult to ensure an even tan since the tan often is not visible until sometime after the cream or lotion has been applied to the skin.

It is also known to have such creams and products applied by a beautician. An advantage of this method is that a more even tan is usually achieved.

More recently it has been known to apply a sunless tanning lotion to the skin of a person with an air brush. This known method involves a beautician applying a sunless tanning lotion to a client using an air brush to spray the sunless tanning product over the skin of the client to achieve an even coverage over the skin.

A problem with this existing method is that although tanning products are not harmful when inhaled, beauticians applying the product to clients may be exposed over significant periods of time to particles of sunless tanning lotion which may "hang" in the atmosphere during and after the air brushing process. In addition, a client to which the product is applied is often surrounded by a mist of the tanning product during and after the spraying process and so may inhale a significant quantity of the product during the tanning process. This can cause discomfort.

Another problem with known methods and apparatus for applying sunless tanning lotions onto a person, is that if the atmosphere within a booth in which the application takes place becomes comfortably warm, the person may sweat causing streaking of the sunless tanning lotions.

A further problem with known methods and apparatus for applying sunless tanning lotion onto a person, is that once the product has been applied to the person, liquid waste is created which must be removed from the booth.

European patent application no. EP 1 238 642 discloses an apparatus for the coating of a human body with a tanning composition, such as a sunless tanning composition, wherein an arm with a plurality of nozzles thereon traverses within a booth to spray coat a body in the booth. The spray nozzles are oriented to avoid opposing air flows and excessive air flows which cause inefficient and uneven dripping or streaking in the deposition of tanning composition on the skin.

A tanning booth is described in our co-pending International patent application of even date.

According to a first aspect of the present invention there is provided a booth for accommodating a person, the booth defining a booth volume and comprising:
a base portion and a top portion;
flow means for causing air to move in a downward air flow within the booth, the downward airflow defining a predetermined volume within the booth;
recirculating means for recirculating the air within the booth;
filtering means for filtering the air within the booth; and
projecting means for projecting a product into at least some of the booth volume and onto a body of a person positioned in the booth, characterised in that the flow means comprises a first plenum of positive pressure located in the top portion of the booth, and a second plenum of negative pressure located in the base of the booth, one or more air input grills associated with the first plenum and one or more foot grates associated with the second plenum, and in that the first and second plenums are connected to one another via a duct, and in that the filter means comprises a first filter for filtering wet particulate material; and a second filter for filtering dry particulate material, wherein the first filter is positioned within the duct in a lower portion of the duct associated with the second plenum and the one or more foot plates, and the second filter is associated with one or more input grills.

The booth is adapted to accommodate a person requiring a product to be applied to her skin. The downward air flow within the booth means that any excess product projected within the booth, which does not adhere to the body of a person positioned within the booth, is carried downwards in the air flow. This means that fewer particles of the product remain in the atmosphere and therefore fewer particles will be inhaled by either the person receiving the tan, or the beautician, or both.

Preferably, the booth further comprises temperature means for controlling the temperature of air circulating within the booth.

The combination of a downward air flow and temperature means for maintaining the temperature within the booth at a predetermined temperature helps in drying the body of a person to which the product is to be applied.

Preferably, the temperature means maintains the temperature within the booth at around 29°C to 30°C. The inventors have discovered that if the temperature is maintained at this level, then vasodilatation occurs in the blood capillaries of the person receiving the product. It is believed that this causes increased blood flow close to the skin, which increases the skin's ability to absorb fluids exterior to it. This reduces the time taken to apply product to a person, and results in a quicker drying time. When the product is a sunless tanning product, this results in a deeper and longer lasting tan.

If a person were to stand in a 29 to 30°C environment with static, or slow moving air, rather than with a downward air flow, there would be a high risk that the person would perspire. If a person to which the product is to be applied perspires, then the product may streak due to it being disturbed and redistributed unevenly on the skin prior to absorption. However, due to the downward air flow moving over the skin of the person standing in the booth, the person either does not perspire, or perspires at a very low level.

The combination of the downward air flow and the temperature means thus results in perspiration being significantly reduced or prevented and exposes the skin to a higher than ambient temperature. This assists in the absorption of the product, without experiencing the usual attendant problems that occur in an overly warm environment.

Alternatively, the temperature means maintains the temperature at 33°C.

The downward air flow also causes product that does not adhere to the skin of a person to be drawn to a lower surface in the booth. This reduces the amount of excess spray particulate which comes into contact with inner side surfaces of the booth, which in turn, reduces the frequency at which it is necessary to wipe down the booth.
The filtering means is positioned so that excess product not adhering to the body of the person in the booth passes through the filtering means in order that both wet and dry particulate are completely or substantially removed. The filtering means ensures that no, or very little, liquid waste is produced, and therefore it is not necessary to drain liquid waste from the system. This in turn, means that the booth does not have to be connected to any external services, and also, removes, or significantly reduces the risk of legionnaire's disease propagating within the waste liquid.

The booth is preferably used to apply a cosmetic product to the skin of the user. The cosmetic product could be any of a range of products such as skin moisturisers, but preferably the cosmetic product is a sunless tanning product.

The flow means comprises a first plenum of positive pressure located in the top portion of the booth, and a second plenum of negative pressure that is located in the base portion of the booth. The first and second plenums therefore result in a downward flow of air from the first plenum towards the second plenum.

Preferably, the first plenum pressurises air to a pressure in excess of ambient pressure, and the second plenum depressurises air to less than atmospheric pressure.

Advantageously, the upper plenum inputs 10 to 15 % less air into the booth volume than the lower plenum draws from the booth volume. This results in air flowing into the booth through any parts of the booth that are open to its surroundings.

The booth comprises one or more air input grills associated with the first plenum and one or more foot grates associated with the second plenum.

In use, air enters the booth through the one or more air input grills, or any other parts of the booth that are open to the surrounding atmosphere. The differential pressure between the first and second plenums causes the downward flow of air which is directed to the one or more foot plates associated with the second plenum.

Preferably, the recirculating means comprises any suitable air moving device such as a fan. The air moving device is used to maintain the pressure differential between the first and second plenums and therefore contributes to the downward flow of air within the booth.

The first and second plenums are connected to a duct in which the air moving device is located. The first and second plenums, the duct, the air moving device and the booth volume together form an air management system.

Preferably, the duct comprises a plurality of duct sections.

The booth may be considered as two volumes of air connected by the air mover.

Advantageously, the recirculating means has a maximum cross-sectional dimension, and the duct has a maximum cross-sectional dimension which is substantially greater than the maximum cross-sectional dimension of the recirculating means.

The duct can thus be used for more than one purpose, as the cross-sectional area of the duct far exceeds that which is necessary to allow air flow around the booth.

The filter means may be placed within the duct, preferably in a lower portion of the duct associated with the second plenum and the one or more foot graters.

This means that excess spray directed through the foot grate by the downward air flow will pass through the filter means.

The filter means comprises a first filter for filtering wet particulate material.

Any suitable material may be used but preferably, the first filter is a high impedance filter.

Conveniently, the first filter comprises polyester material that filters fine particulate and wet particulate, such as a polyester fibre weave.

The filter may also, or alternatively be placed in association with the first plenum and the input grill.

Advantageously, the filter means comprises a third filter which is a low impedance filter, which serves to filter excess wet particulate material after the spray has passed through the first filter. The second filter also acts to ensure that air flow is distributed substantially evenly across the foot grate.

Once wet particulate has been captured in the first and third filters it dries, due to the air flow within the booth. This ensures that little, if any, liquid waste is produced, and therefore ensures that the booth does not have to be connected to external services.

Conveniently, the second material comprise "andrea" type perforated concertina card filter.

Preferably, the filter means comprises a fourth filter for filtering any remaining dry particulate.

Any suitable filter may be used, but advantageously the fourth filter is one that is conventionally regarded as coarse and with a low air movement impedance. However, such a filter is conventionally intended to have a depth of 100mm. The inventors have discovered that by making such a filter with a depth of approximately 750mm, air movement is not greatly impeded, but air has to pass though seven times as much course media than is conventionally the case. This not only dramatically increases the efficiency of the filter in removing dry particulate, but also lends considerable sound dampening characteristics to the movement of large volumes of air through the booth.

Conveniently, the fourth filter comprises a loose bonded fibre filter.

The filter means comprises a second filter associated with the one or more input grills. The impedance of the second filter is preferably varied across the filter to ensure that the air distribution is encouraged towards the front of the air input system. This encourages a venturi effect.

The Venturi Effect (or Bernoulli Effect) states that when the velocity of a fluid (for example, air) is increased its pressure decreases. Therefore, by increasing the speed of air issuing from the air input grill, an area of low pressure is created within the downward air flow issuing from the input grill. This is achieved by pressurising the first plenum and then allowing the pressurised air to escape at speeds typically approximately 1.2ms⁻¹. Due to the fact that the ambient atmospheric pressure will be greater than the pressure within the downward air flow issuing from the input grill (lowered according to Bernoulli's Principle), air will move from outside the booth volume to attempt to equalise this pressure differential. This results in a constant flow of fresh air coming into the booth volume from portions of the booth that are open to the surroundings.

The Bernoulli effect, combined with pressure differentials existing within the booth due to the relative overpressure in the first (upper) plenum and the relative underpressure in the second (lower) plenum, result in an inflow of air from outside the booth volume into the booth volume. This effect, in combination with the effect of the input grill or grills which typically have a concave shape, means that the incoming air can be used to increase the air speed within the booth volume particularly towards a central portion of the booth volume. This leads to advantages in terms of extract rate of product, and the energy efficiency of the air mover. In addition, due to the high airspeed of the downward flow of air, product is less likely to be inhaled by a person using the booth.

Conveniently, the second filter comprises a polyester fibre weave fine particulate filter.

The relatively high impedance of the fourth filter ensures that sufficient air pressure is generated within the first plenum to cause an appropriate proportion of air volume to be forced out of the booth through, for example, bleed sites formed in the booth.

Optionally, the filter means further comprises a fifth filter which may be in the form of a two-part filter, the first part comprising a mechanical particulate filter, and the second part comprising an electrostatic charge filter.

Conveniently, the fifth filter comprises loose bonded fibre filter and an electrostatically charged needle perforated synthetic sheet filter.

By means of the present invention, a downward air flow having a high air speed is directed down towards a vertically central portion of the booth volume, to define the predetermined volume.

Preferably, the air speed is 1.2ms⁻¹ towards a central portion of the booth volume, and above 0.8ms⁻¹ in an outer third of the booth volume, when a client is not inside the booth volume. When a person is inside the booth volume, thereby occupying some of the volume, the air speed increases within the booth volume. This results in a more even distribution of air speed within the booth volume of 1 and 1.2ms⁻¹ throughout the booth volume. However, this is a figure which is representative, and there are areas within the booth volume in which the air speed can be as low as 0.7ms⁻¹, depending on exactly where the person stands within the booth volume. It is in this portion of the booth that a person to whom the product is to be applied will stand. The increase in air speed adjacent the skin of the person is therefore also high. This ensures that any excess particulate sprayed onto the person which does not adhere to the skin is extracted more speedily and with negligible loss of excess particulate from the booth volume prior to entering the filter means.

The projecting means may comprise a hand held tool such as an air gun. The air gun allows an operator to utilise a small hand held spraying tool to spray a sunless tanning product onto the skin of a client. However many different products may be sprayed through the unit.

Alternatively the hand held tool could be an airless sprayer.

Advantageously, the predetermined volume defined by the downward air flow comprises a portion only of the booth volume.

This means that a beautician or other operator applying the sunless tanning lotion to a person may stand outside of the predetermined volume when applying the lotion to the client. This reduces the exposure of the operator to the tanning lotion.

Similarly, a person may leave personal belongings in a portion of the booth situated outside of the predetermined volume, which belongings will therefore not be exposed to the particles of sunless tanning lotion.

Preferably however, the projecting means comprises a remotely operated tool.

Advantageously, the remotely operable tool comprises a plurality of nozzles that project the product into the predetermined volume. This means that when a person is standing in the predetermined volume defined by the downward air flow, the remotely operated tool will direct a spray of product onto the client.

Preferably, the remotely operated tool comprises adjustment means for adjusting the height of the nozzle. This means that a person may stand within the predetermined volume while the height of the nozzle is adjusted in order to ensure complete and even coverage of the body of the client by the product.

Preferably, the adjustment means additionally adjusts the attitude of the nozzle. This means that the angle at which the product is incident on the body of a person may be varied in order to ensure consistent overall coverage of the product on the person's skin.

In a preferred embodiment of the present invention, the remotely operable tool comprises means to automatically transport the tool to provide spraying between two zones in the booth. Preferably, the two zones comprise substantially the entire height of the booth.

In one form, the automatic transport means comprises at least one slider unit moveable vertically between two positions, the slider unit supporting at least one spray means.

Preferably there is provided two slider units supporting an arcuate spray arm comprising a plurality of spray guns.

The spray guns may be directed to spray product horizontally and/or at an angle to the horizontal (whether upwardly or downwardly) and/or some combination of these.

Preferably, the control system operates the projecting means to project specified amounts of the product in selected regions of the booth volume, the specified amounts varying from zero to maximum flow of the product from the projecting means.

Preferably, the projecting means is movable along a path within the booth and the control system operates the projecting means as it moves along the path in accordance with predetermined instructions.

There is also provided a computer program product directly loadable into the internal memory of a digital computer, comprising software code portions for performing the steps of the method.

According to a second aspect of the present invention there is provided a tool for projecting a product into a booth, the booth comprising a base portion and a top portion, and flow means for causing a downward air flow within the booth.

According to a third aspect of the present invention there is provided a method for applying a product to a human body comprising the steps of:
causing air to flow in a downward direction onto the human body within a booth, the booth defining a booth volume and comprising a base portion and a top portion;
flow means for causing air to flow in a downward air flow within the booth, the downward air flow defining a predetermined volume within the booth;
recirculating means for recirculating the air within the booth;
projecting means for projecting a product into at least some of the booth volume onto the human body, the flow means comprising a first plenum of positive pressure located in the top portion of the booth, and a second plenum of negative pressure located in the base of the booth, one or more air input grills associated with the first plenum and one or more foot grates associated with the second plenum;
recirculating the air flowing onto the human body;
filtering the air to remove wet and dry particulate material;
projecting a product onto the human body, wherein the step of filtering the air to remove wet and dry particulate material comprises passing the air through a first filter for filtering wet particulate material;
passing the air through a second filter for filtering dry particulate; characterised in that:
   the first filter is positioned within the duct in a lower portion of the duct associated with the second plenum and the one or more foot plates, and the second filter is associated with one or more input grills.

Preferably, the method comprises the further step of controlling the temperature of the air flowing onto the human body.

The invention will now be further described by way of example only with reference to the accompanying drawings in which;
Figures 1a and 1b are schematic representations of two booths according to a first embodiment of the present invention;
Figure 2 is a schematic representation of a second embodiment of a booth according to the present invention;
Figure 3 is a schematic representation of a projecting means forming part of the booth of Figure 2;
Figures 4a, 4b and 4c are schematic representations of a variation of the booth of Figure 1;
Figures 5a, 5b and 5c are schematic representations of a variation of the booth shown in Figure 2;
Figure 6a and 6b is a schematic representation of booths according to the invention showing the air flow within each booth;
Figure 7 is a schematic representation of a booth shown in Figures 1a or 1b showing the air flow in more detail;
Figure 8 is a flow chart showing how air is recirculated within a booth according to the present invention;
Figures 9 to 12 show various forms of spray ring forming part of the booth of Figure 2;

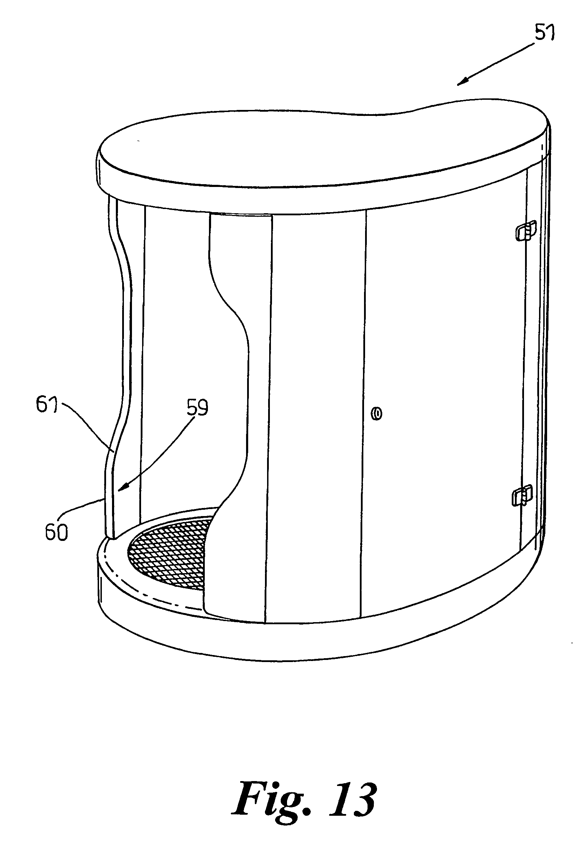

Figure 13 shows the exterior of the booth of Figure 2; and
Figure 14 shows the door arrangement of the booth of Figure 2.

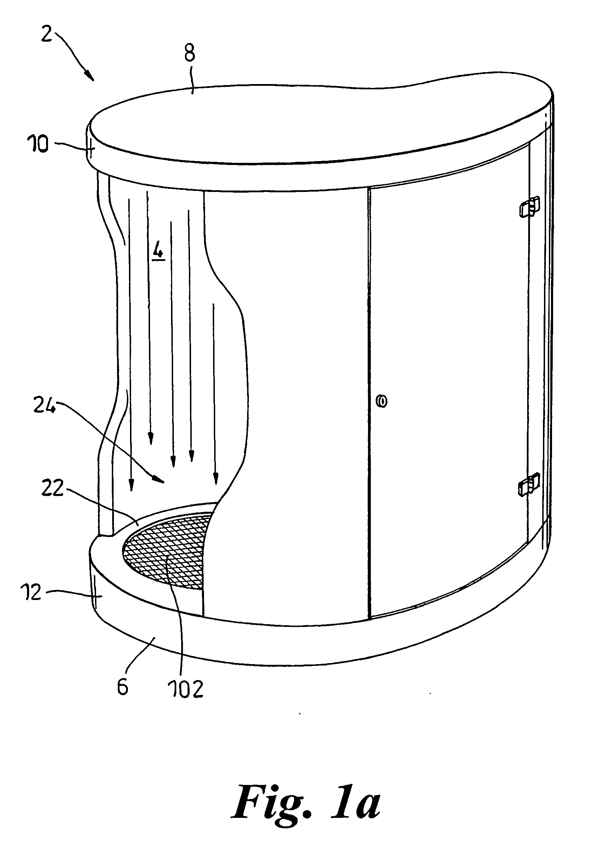

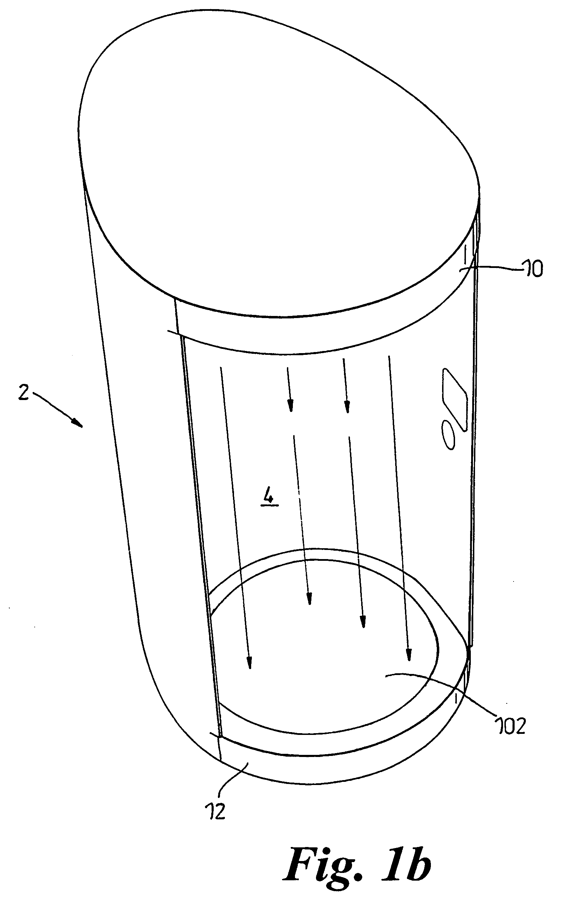

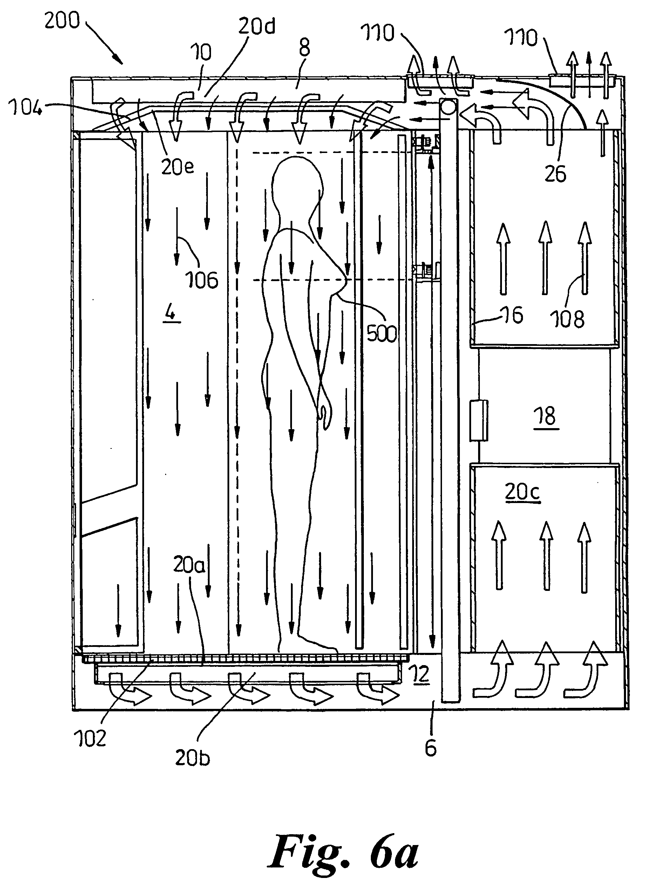

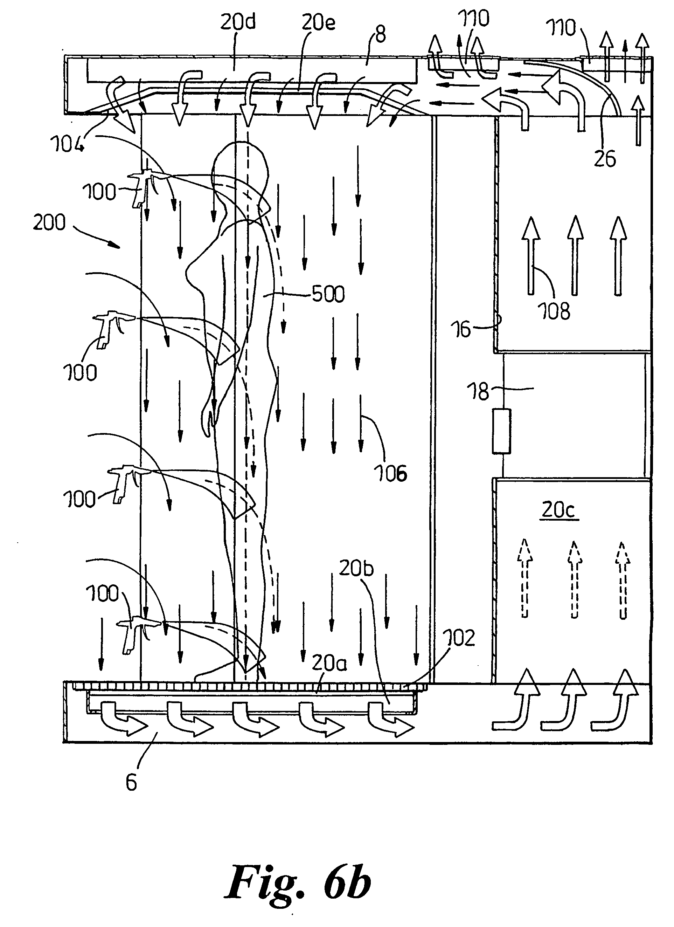

Referring to the figures, a first embodiment of a booth according to the present invention is shown in Figures 1a and 1b is designated generally by the reference numeral 2. The booth defines a booth volume also known as a spray chamber 4 and comprises a base portion 6 and a top portion 8. The booth 2 comprises an upper plenum 10, and a lower plenum 12. The upper plenum has a positive pressure with respect to atmospheric pressure, and the lower plenum has a negative pressure with respect to atmospheric pressure. This pressure difference causes a downward flow of air in the direction of arrows 14 (see Figure 6) of air circulating within the booth 2. In this embodiment, the upper plenum draws in 10 to 15% less air into the booth volume than the lower plenum draws from the booth volume. The booth 2 further comprises a duct 16. Positioned in the duct 16 is an air mover 18 in the form of, for example a fan. The air mover 18 allows air to be recirculated within the booth 2.

The booth further comprises filters 20, the purpose of which will be described further herein below.

The booth 2 is used to enable a person 500 such as a client of a beauty salon to obtain an even tan through application of a sunless tanning lotion applied by an operator using an air gun.

The person 500 to be tanned stands on the floor 22 of the booth 2 within a predetermined volume defined by the downward flow of air. The floor 22 comprises a foot grate 102, the purpose of which will be described in more detail below. An operator using an air gun 100 may stand at the operator access opening 24, which is outside the predetermined volume. The spray gun is attached to a supply of the tanning product to be applied to the client's skin. The spray gun causes the product to be sprayed onto a client's skin.

By means of the present invention therefore a highly specific and controlled delivery of atomised products such as a sunless tanning product may be applied to the client. There is no need for the client or the operator to manually manipulate the product once it has been applied to the skin in order to ensure even coverage.

During the act of spraying, the tanning product becomes air borne in the form of small droplets. The downward air flow of the present invention ensures that any excess small droplets that do not adhere to the skin of the client, are kept away from the client and the operator, and that a clean supply of air for both the operator and the client is available. The filters 20 serve to filter out particles of the product to ensure that the recirculating air is as clean as possible.

Although there is a need for operator access, the downward air flow prevents air loss via the operator access opening 24. A percentage of the air flow is bled off from the first plenum 10 in order that the differential between the air volume being drawn from the booth volume 4 into the second plenum and the air volume being moved into the booth volume 4 from the first plenum 10 is replaced by air moving into the downward flow of air within the booth volume from outside the booth 2 via the operator access opening 24. This management of the air flow prevents or minimises any outward flow of particles from the booth volume 4 during the spraying process and ensures that all particles generated within the booth volume 4 pass through the filters 20 thus ensuring that the client and the operator are maintained in clean air.

A downward flow of air is the most efficient way to move air over a human body as this ensures that the body presents a minimum cross sectional area of impedance to the air flow.

The downward air flow capitalises on the tendancy for heavy atomised particles to drop downwards under the influence of gravity. The downward air flow thus uses gravity to assist in the process of extracting particles generated by the spraying tool from the booth volume 4.

Since air is recycled within the booth 2, it will not be necessary for a purchaser of the booth 2 to carry out building work to their premises in order to create ductwork to the atmosphere. By using a recirculating air stream there is no need for any building work to be carried out since no ductwork to the atmosphere is required.

The booth 2 further comprises a heater 26 which warms the air in the downward air flow. The combination of spraying a wet product onto a client's skin in conjunction with an air stream moving over the body of a client creates a "chill factor". By means of the heater 26 it is possible to ensure that air passing over the body of a client is at an appropriate temperature. The temperature of the air is kept at around 29-30°C. As explained hereinabove, this temperature causes vasodilatation in the blood vessels of the person in booth. This increases absorption of the product sprayed onto the person. In addition, the downward air flow reduces or prevents perspiration by the person standing in the booth. Further, after the spraying process has been completed, a client may be dried by the air flow.

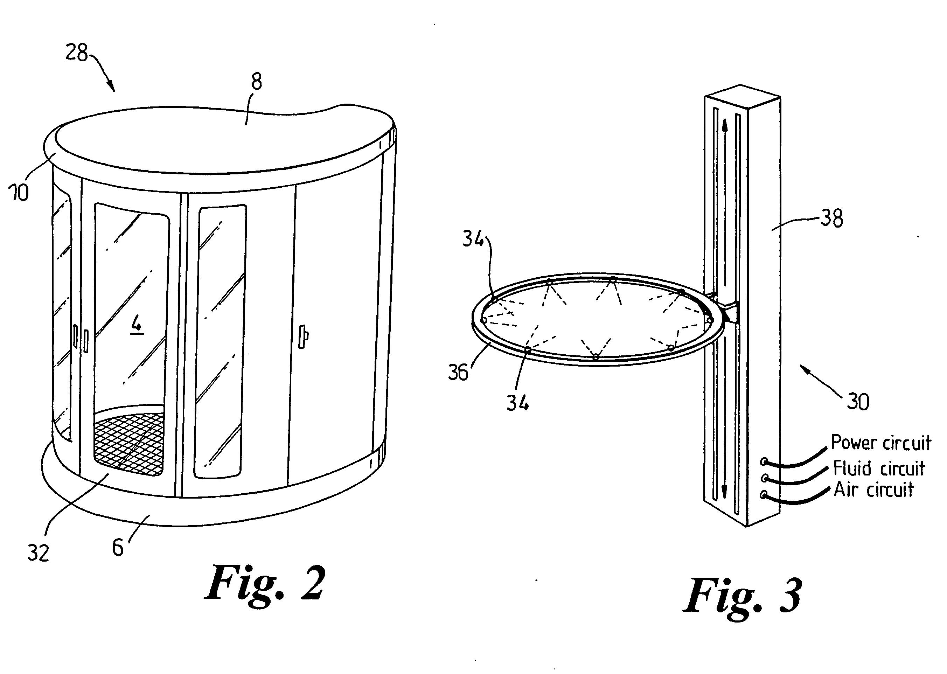

Turning now to Figures 2 and 3, a second embodiment of the invention is designated generally by the reference numeral 28. For all parts which correspond to parts shown in Figure 1, the same reference numerals have been used for clarity. In addition, the air flow within the booth 28 is similar to that within the booth 2 shown in Figure 1, and Figure 6 also relates to the air flow within the booth 28.

Booth 28 comprises a remotely operable tool 30 (shown in Figure 3) for applying a sunless tanning product to a client. The booth is similar to the booth shown in Figure 1 but additionally comprises doors 32 which may be closed once a client has entered the booth 28. The tool 30 comprises a plurality of nozzles 34 supported by a nozzle support 36 which is generally circular in shape. The nozzle support 36 is mounted on a post 38 which is attached to a power circuit, fluid circuit and air circuit forming part of the booth 28.

The tool 30 is an automated system which allows a client to be sprayed with a sunless tanning lotion, for example, without the need of an operator. The nozzle support 36 is the form of a lasso which provides mounting points for several nozzles 34 around its perimeter. The lasso then descends over the client standing in the area of the down flow of air and the nozzle delivers the product to be applied to the client in a metered manner. The tool 30 also contains means for adjusting the angle of the nozzles in order that each nozzle may move through an arc pointing alternately upward and downward to vary the angle of incidence of the product on the client during the spray cycle. This ensures that the client's skin receives a comprehensive coating of the product and further removes the necessity for manually manipulating the product once on the skin. The client remains in a static position during the spraying process. Following the spraying process the client remains within the heated recirculating air flow in order to allow the product to completely and quickly be absorbed by and dried upon the skin by the heated recirculating air flow.

A further advantage of the tool 30 is that once the lasso 36 has moved below the head of the client, the downward air flow ensures that a minimum amount of spray will come into contact with the client's face. The client is therefore able to breath freely during almost all of the spraying process.

The booth 28 comprises bleed vents (not shown) to enable air to be drawn into the booth during operation of the booth.

Although the invention has hitherto been described with reference to a heating apparatus for heating the air flowing through the booth, it is envisaged that in warmer climates or during the summer of temperate climates it may be advantageous to cool the air flow.

It will also be possible to "upgrade" the version of the booth shown in Figure 1 in which an operator is required to apply the product to a client to the version of the booth shown in Figure 2 in which the product is applied automatically. This could be done by installing tool 30 into booth 2, and fitting doors to the booth.

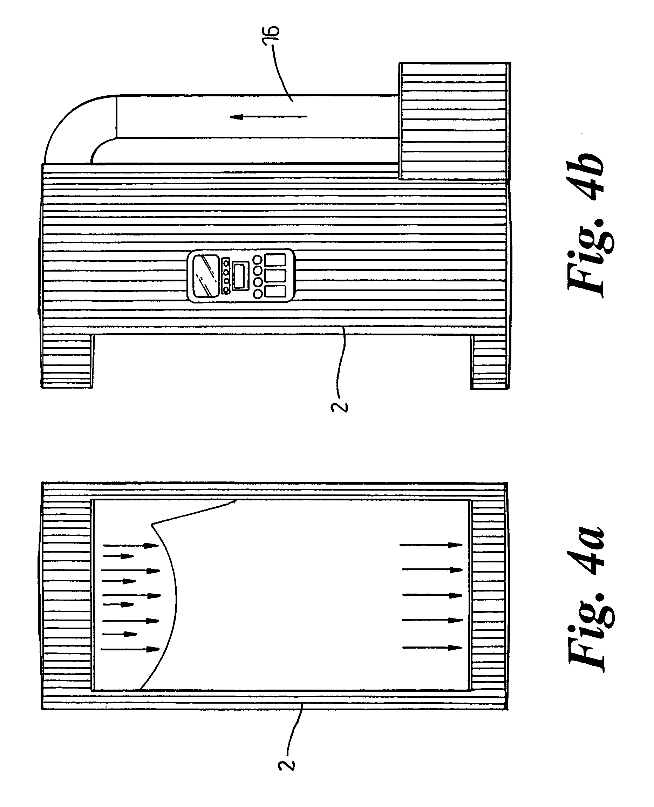

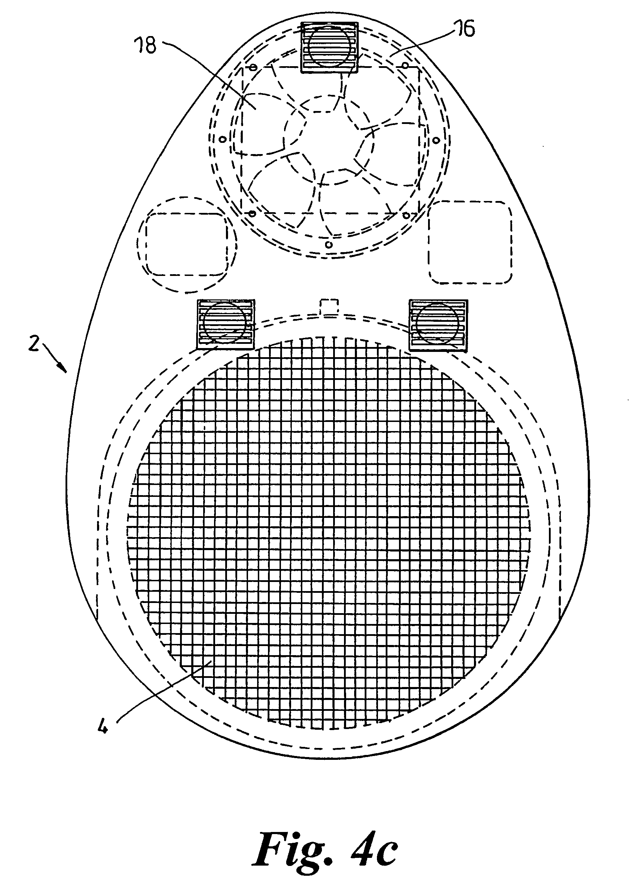

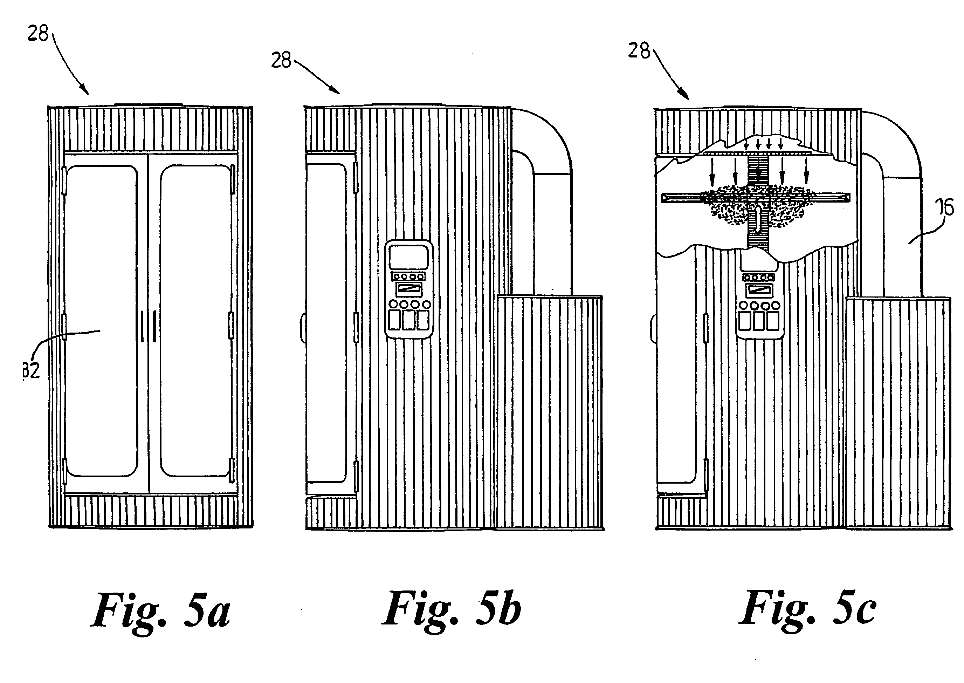

Referring to Figures 4a, 4b and 4c a variation of the booth in Figure 1 is shown. Similarly Figures 5a, 5b and 5c show a variation of the booth shown in Figure 2.

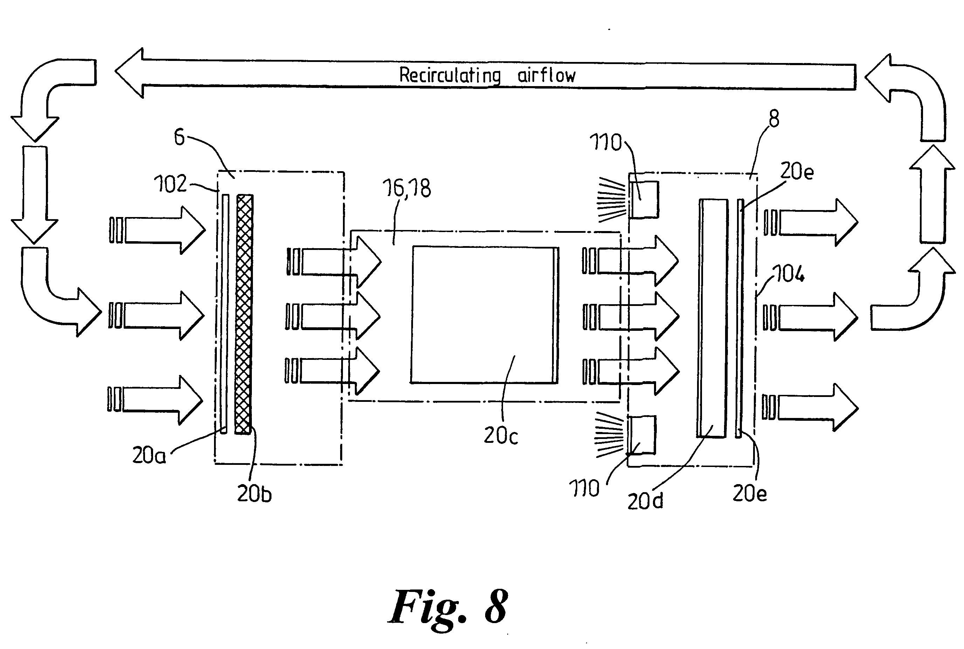

Referring now to Figures 6a, 6b and 8, the air management system of a booth according to the present invention will be described in more detail. Components which correspond to components forming part of the booths described hereinabove have been given corresponding reference numerals for ease of reference.

The air management system of a booth 200 comprises duct 16, air mover 18 in the form of, for example, a fan, upper plenum 10, lower plenum 12 and spray chamber 4. The upper plenum includes an air input grill 104, and the lower plenum includes a foot grate 102. The booth 200 further comprises filters 20 which serve to remove all or substantially all of any wet or dry particulate sprayed into the spray chamber 4, that does not adhere to the body of the person standing in the chamber. The booth 200 further comprises a heater 26, which in this embodiment maintains the temperature of recirculating air at approximately 29 - 30°C.

The first plenum 10 is kept at a pressure above atmospheric pressure, and the second plenum 12 is kept at a pressure below atmospheric pressure. This causes a downward flow of air as indicated by the arrows 106. The air is recirculated and passes through a first filter 20a that catches a majority of any excess wet spray, hair, and large skin flakes which are directed downwardly by the downward air flow and through the foot grate 102.

The air then passes through a third filter 20b, which serves to collect substantially all of any remaining wet particulate being carried by the air.

The air is then directed up the duct 16 in the direction of arrows 108. The air passes through a fourth filter 20c which collects substantially all remaining dry particulate, crystalline particulate and dust remaining in the air.

Once the air has reached the top of the duct 16, a portion of the air will exit the booth 200 via filtered pressure relief ports 110. Air not existing at this point, is directed through the air input grill via a second filter 20d which is a two part filter used for collecting remaining dry particulate, crystalline particulate and dust. Finally, the air passes through a fifth filter 20e, which is used for collecting remaining crystalline particulate and dust.

By means of the present invention substantially all waste is removed from the system thus ensuring that the booth can operate as a stand alone unit without having to be connected into any waste disposal systems.

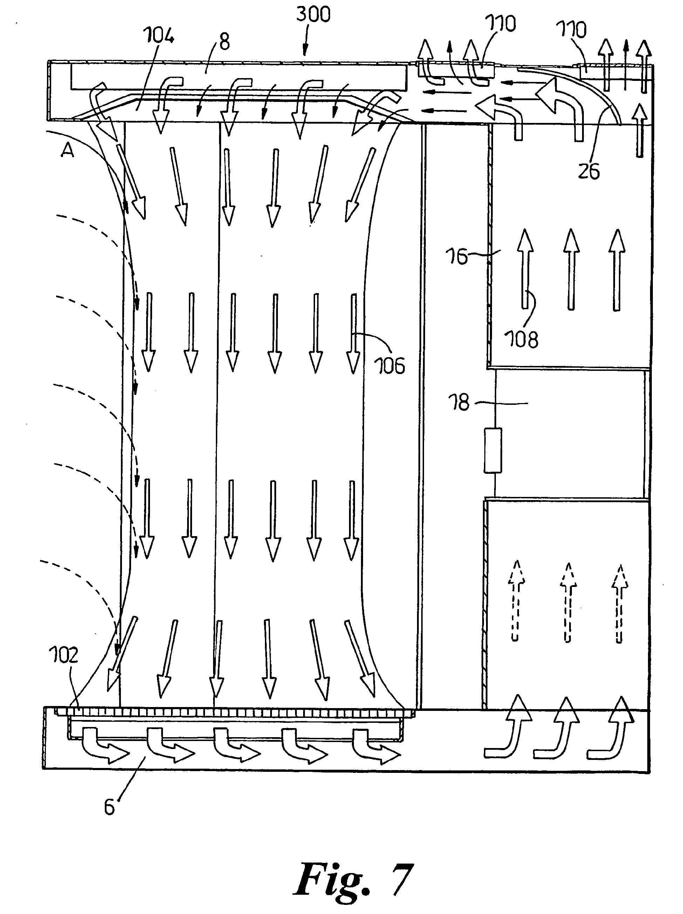

Referring now to Figure 7, air flow in a manual booth 300 of the type shown in Figures 1a and 1b is shown. It can be seen that due to the design of the air input grill 104, a venturi effect is developed which encourages air to be drawn in through the operator access opening.

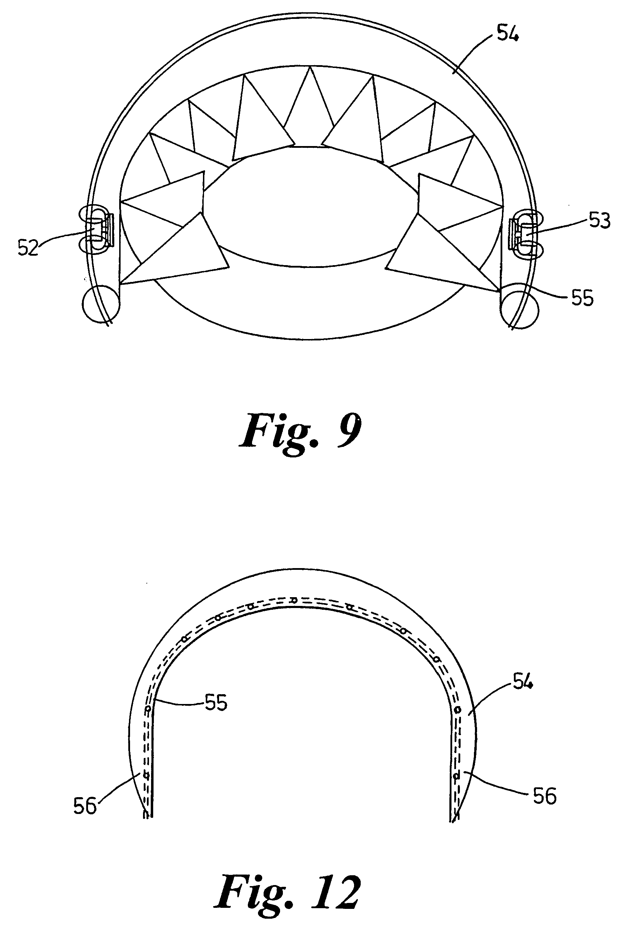

There is shown in Figure 9 part of a spraying mechanism 50 of a further embodiment of booth 51.

The interior of booth 51 contains two slide units 52 and 53 which support a spray arm 54 to provide vertical upward and downward movement of arm 54 over the entire height of the booth.

Spray arm 54 comprises a horizontal arcuate arm containing a plurality of spray guns 55 to direct tanning lotion inwardly and horizontally generally towards the centre of arm 54 and hence onto any occupant of the booth.

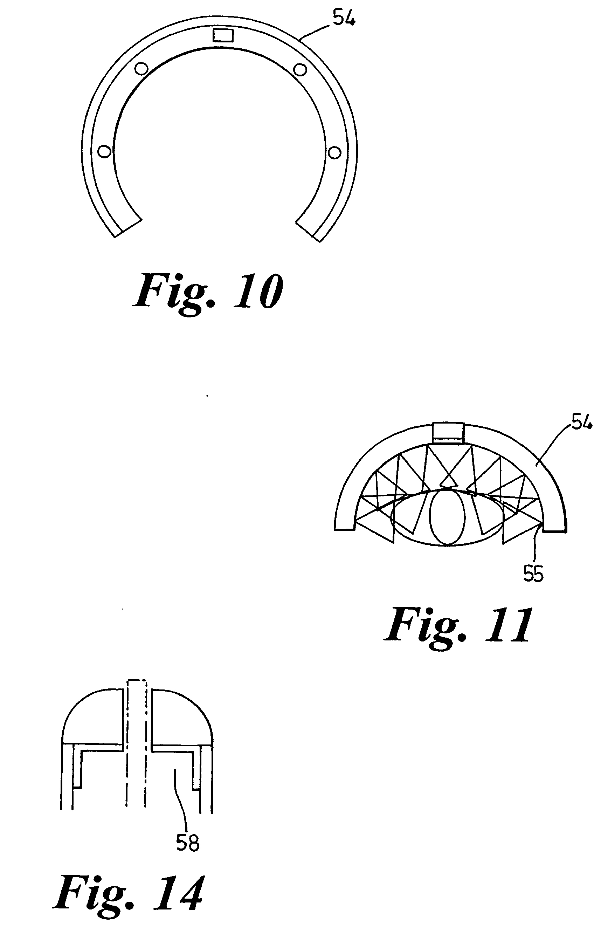

Typically, spray arm 54 is of semi-circular form and slide units 52 to 53 are located at diametrically opposing positions on the periphery, as shown in Figure 7, but various exemplary variants are shown in Figures 9 to 11.

Thus, spray arm 54 can be shaped to form an arc of from 150° to 270° of a circle, and may include straight end portions 56 beyond the arc as shown in Figure 12. The spray guns may extend along the entire periphery of the arm.

In a variant, the slide units are not diametrically opposite on the periphery, but may be closer along the periphery, for example they may be at points on the periphery defining an angle of 90° therebetween.

The spray guns are positioned so as to direct the lotion horizontally. In a variant, they may be positioned to direct the lotion at a small angle inclined to the horizontal. In another variant, the guns may be positioned to direct the lotion at various different angles either upwardly or downwardly, and either in a random manner or in an organised, progressively changing manner.

Spraying mechanism 50 also has a motor 57 and a gearbox 58 to provide the vertical motion of slide units 52, 53.

The "open" configuration of spray arm 54, as compared to the closed circular form of lasso support 36, has been developed with safety in mind. The spray arm 54 allows client access in from one side, regardless of the position within the spray chamber and it enables a quick exit in case emergency procedures need to be followed. It also allows for a quick exit if the client, for whatever reason, feels uncomfortable with the process. If movement of the spray arm 54 is halted at any position during its travel, the client is still able to exit the booth with minimal discomfort or danger.

In order to spray the client over the whole body using this design of spray arm 54, the application of tanning lotion must be applied in two steps. In the first step the front section of the body is sprayed; the client then turns through 180 degrees, and actuates operation of the spray arm 54 again, thereby applying lotion to the rear section of the body. Multiple spray guns would only be seen on one section the spray arm.

The spray mechanism 50 of booth 51 has an operation control facility allowing a client to programme operation of the slide system and spray arm 54 to accommodate their specific requirements. For example, the client may wish to have only the face sprayed, or only the face and shoulders, or only top sections of the body, or only lower section of the body, or indeed the entire body.

This system makes provision for this by allowing the client to select where the spray guns switch on and off during vertical travel of the spray.

In order to programme such a sequence, the client operates a series of push buttons on a remote hand-held-device during a slow, practice sequence of the slide system. As the slide moves along the vertical path, for example the "guns-on" position is set when appropriate, followed by the "guns-off" position when appropriate.

Once the operation has been selected, the slide is returned to the rest or start position, allowing a full spray cycle to occur once the operator subsequently presses the start button. The system allows each of a number of different programs to be stored, so that any can be re-used if or when appropriate. The system allows cancellation of the programmed switching points, enabling re-programming of the switching points.

Booth 51 has a motor drive system which allows for programmable and controlled movement of the spray arm 54 throughout the vertical travel.

Alternative designs may allow for pneumatically actuated slide systems, but they tend to lack precise speed control, acceleration and deceleration parameters as compared to the programmable, electrically driven slide system.

The drive system is based on a stepper motor arrangement, allowing for the programmable switching of the spray guns according to the different requirements of the client. Alternative drive solutions include servo motor control, AC and DC stepper motors.

In addition to the drive motor arrangement, the rate of rotation is reduced using a gearbox arrangement. This provides a higher torque, required to overcome the weight of the spray arm 54 and actually allows for more precise positioning of the slide systems. If using a system with single slide, only one output shaft is required, directly coupled to the slide input shaft. However, the current design allows for the driving of two slides located opposite each other. Therefore, the gearbox includes two output shafts and single gearbox and two slide systems are connected using flexible drive shafts. These are located within the upper chamber to be closer to the control system, although it is feasible to locate them in the lower chamber if required.

The door arrangement comprises two sliding doors, meeting in the centre and allowing for manual closure of the doors and automated opening of the doors using actuated cylinders following the drying process.

In order to keep the doors closed, a magnetic latch system is used. This allows the doors to stay closed against the spring-loaded "opening" system, but also allows for manual release of the doors should an emergency procedure arise, requiring the client to exit the booth. If a power cut experienced, the doors are automatically opened by default, immediately providing a clear exit for the client.

Alternatively, a mechanical latch system may be used.

Booth 51 has a novel runner system, based on the utilisation of precision bearings and guide rails.

The automatic control panel is fitted within a space, previously provided within the booth 51 and accessible through an equipment access panel located to one side of the main booth structure.

Booth 51 has a compressor arrangement to pressurise a 25 to 75 litre reservoir, 50 litres being the nominal, to a pressure exceeding 4 Bar. The reservoir is used for storing the necessary energy required to supply the multiple automatic spray guns, mounted to the spray arm 54, with atomising air and air required for effecting the Fan Size. In addition, air is used for triggering the spray guns, allowing them to spray fluid. Air will also be used for triggering the door release cylinders, although these may also be done using electronic means, (solenoids).

The booths 2 or 28 as previously described can readily be modified either prior to installation or once at the operational location in order to have the features and operability of the more technically sophisticated booth 51.

Also any booth 51, whether so modified or otherwise, can still be used in a manual operation mode, for example to provide certain additional applications including highlighting and detailing, since some clients may prefer a manual application.

In order to modify a manual tanning booth 2, the following actions need to be taken:

The inner skins of the manual tanning booth 2 are removed in order to gain access to the major re-fitting areas associated with the fitting of slides and the associated drive system.

The two slide units 52, 53 are mounted and secured within the booth structure between the outer skin and the area allocated to the inner skins.

In order to apply lotion along the entire internal height of the booth, it is necessary to "sink" each end of the slide units 52, 53 into the base portion 6 and top portion 8 of chamber 2.

In order to fit the slide units 52, 53, the base and top portions 6, 8 have a pre-machined location feature integrated to the manual booth structure in preparation for easy, on-site upgrades.

Once the slide units 52, 53 are secured in position within the inner section of the tanning booth, cover plates are fitted over the slide(s). Cover plates are required in order to isolate the spray area (contamination area), from the slides. Excess material being deposited on the running features of the slides may prove detrimental to the long running cycles of the system.

These cover plates are integrated in such a way that they may also add to the structural robustness of the system. Once fitted, the spray arm 54 is mounted to the slide carriage plate(s). The slide carriage plates are designed such that they allow for secure fixing to the slide(s), whilst maintaining the isolation between the contamination area (spray area), and the slides by running through bush strips within the cover plates.

Although due to the pressure differentials between the top and bottom plenums and the bleed off from the top chamber, these potential "air leak" sites have air moving through them into the spray chamber.

All services, (electrical, pneumatic and fluid supply), are also managed through the slide area to the spray arm 54 using the same disciplines to prevent contamination from within the spray area to the internal equipment functions within the booth equipment enclosures. This ensures robust, reliable and safe operation of the system to be maintained.

Once the spray arm 54 has been fitted and associated service lines attached, the motor drive system is fitted.

Following the fitting of the spray ring 54 and associated drive system, and whilst access is still maintained to the inner sections between the skins, the sliding front doors are now fitted. These doors, not present on the manual system, are required to enclose the spray chamber. This prevents overspray from leaving the spray booth chamber, and also provides the client with the privacy required to allow the client to be sprayed naked within the booth.

The inner skins are now re-fitted to the inner chamber of the spray booth. These have to be cut in half, and material removed, in order to cater for the fitting of the slide cover plates. Alternatively, new skins are fitted.

In addition, and to accommodate the newly fitted sliding doors, the front vertical aperture supports are replaced with a new design of support simply to accommodate the sliding doors running in between the inner and outer skins. The manual booth supports are removed.

The manual control board is replaced with a fully tested automatic control system capable of running both the manual and automatic elements of the system.

The automatic control panel is fitted within a space, previously provided within the booth enclosure and accessible through the equipment access door located to one side of the main booth structure. This requires relocation of the manual control enclosure, should it be retained within the system, or simply replaced if it is to be removed from the system. Once the control system is fitted, the equipment peripherals are wired up ready for testing.

This completes the upgrade operation.

## Claims

1. A booth for accommodating a person, the booth defining a booth volume (4) and comprising:
a base portion (6) and a top portion (8);
flow means for causing air to move in a downward air flow within the booth, the downward airflow defining a predetermined volume within the booth;
recirculating means (18) for recirculating the air within the booth;
filtering means (20) for filtering the air within the booth; and
projecting means (100;30) for projecting a product into at least some of the booth volume and onto a body of a person positioned in the booth, wherein the flow means comprises a first plenum (10) of positive pressure located in the top portion of the booth, and a second plenum (12) of negative pressure located in the base of the booth, one or more air input grills (104) associated with the first plenum and one or more foot grates (102) associated with the second plenum, wherein the first and second plenums are connected to one another via a duct (16), **characterised in that** the filter means comprises a first filter (20a) for filtering wet particulate material; and a second filter (20d) for filtering dry particulate material, wherein the first filter is positioned within the duct in a lower portion of the duct associated with the second plenum and the one or more foot grates (102), and the second filter is associated with one or more input grills.

2. A booth (2;28) according to Claim 1, further comprising temperature means (26) for controlling the temperature of air circulating within the booth.

3. A booth (2;28) according to Claim 2 wherein the temperature means is adapted to maintain the air flow at about 29°C to 30°C.

4. A booth (2) according to Claim 2, wherein the temperature means is adapted to maintain the air flow at about 33°C.

5. A booth (2) according to Claims 2, 3 or 4 wherein the temperature means comprises a heater.

6. A booth (2) according to any one of the preceding claims wherein the first plenum (10) is adapted to pressurise air to a pressure in excess of ambient pressure, and the second plenum (12) is adapted to depressurise air to less than atmospheric pressure.

7. A booth (2) according to any one of the preceding claims wherein the duct (16) comprises a plurality of duct sections.

8. A booth (2) according to any one of the preceding claims wherein the recirculating means comprises a fan.

9. A booth (2) according to any one of the preceding claims, wherein the recirculating means is located within the duct.

10. A booth (2) according to Claim 9 wherein the recirculating means has a maximum cross-sectional dimension, and the duct has a maximum cross-sectional dimension which is substantially greater than the cross-sectional dimension of the recirculating means.

11. A booth (2) according to any one of the preceding claims wherein the filter means comprises a third filter (20b) comprising a low impedance filter for filtering wet particulate material.

12. A booth according to any one of the preceding claims wherein the filter means comprises a fourth filter (20c) comprising a second low impedance filter for filtering dry particulate material.

13. A booth according to any one of the preceding claims wherein the second filter (20d) has an impedance that varies across the filter.

14. A booth according to any one of the preceding claims wherein the filter means comprises a fifth filter (20e) comprising a first filter component comprising a relatively high impedance filter, and a second filter component comprising a combined mechanical and electrostatic filter.

15. A booth according to any one of the preceding claims wherein the predetermined volume comprises a portion of the booth volume.

16. A booth according to any one of the preceding claims wherein the projecting means comprises a hand held tool (100).

17. A booth according to Claim 16 wherein the hand held tool comprises an air gun (100).

18. A booth according to Claim 17 where the hand held tool comprises an airless sprayer.

19. A booth according to any one of Claims 1 to 15 wherein the projecting means comprises a remotely operable tool (30).

20. A booth according to Claim 19 wherein the remotely operable tool comprises a plurality of nozzles (34) adapted to project the product into the predetermined volume.

21. A booth according to Claim 19 wherein the remotely operable tool further comprises adjustment means (52,53) for adjusting the height of the nozzles.

22. A booth according to Claim 21 wherein the adjustment means additionally adjusts the attitude of the nozzles.

23. A booth according to any one of Claims 19 to 22 wherein the remotely operable tool (30) comprises a nozzle support (54) defining a substantially arcuate shape, the nozzles being positioned to spray the product into an area defined by the nozzle support.

24. A booth according to any preceding claim comprising a remotely operable tool (30) with means to automatically transport the tool to provide spraying between two zones in the booth.

25. A booth according to Claim 24 wherein the automatic transport means comprises at least one slider unit (52,53) moveable vertically between two positions, the slider unit supporting at least one spray means.

26. A booth according to any preceding claim comprising spray guns which are directed to spray product horizontally and/or at an angle to the horizontal (whether upwardly or downwardly) and/or some combination of these.

27. A method for applying a product to a human body comprising the steps of:
causing air to flow in a downward direction onto the human body within a booth (2), the booth defining a booth volume (4) and comprising a base portion (6) and a top portion (8);
flow means for causing air to flow in a downward air flow within the booth, the downward air flow defining a predetermined volume within the booth;
recirculating means (18) for recirculating the air within the booth;
projecting means (20) for projecting a product into at least some of the booth volume onto the human body, the flow means comprising a first plenum (10) of positive pressure located in the top portion of the booth, and a second plenum (12) of negative pressure located in the base of the booth, one or more air input grills (104) associated with the first plenum and one or more foot grates (102) associated with the second plenum the first and second plenums being connected to one another via a duct (16);
recirculating the air flowing onto the human body;
filtering the air to remove wet and dry particulate material;
projecting a product onto the human body, **characterised in that**: the step of filtering the air to remove wet and dry particulate material comprises passing the air through a first filter (20a) for filtering wet particulate material;
passing the air through a second filter (20d) for filtering dry particulate; wherein
the first filter is positioned within the duct in a lower portion of the duct associated with the second plenum and the one or more foot grates (102), and the second filter is associated with one or more input grills.

28. A method according to Claim 27 comprising the further step of passing the air through a third filter (20b) comprising a low impedance filter for filtering wet particulate material.

29. A method according to Claim 27 or Claim 28 comprising the further step of passing the air through a fourth filter (20c) comprising a second low impedance filter for filtering dry particulate material.

30. A method according to any one of Claims 27 to 29 comprising the further step of passing the air through a fifth filter (20e) comprising a first filter component comprising a relatively high impedance filter, and a second filter component comprising a combined mechanical and electrostatic filter.

31. A method according to any one of Claims 27 to 30 further comprising the step of controlling the temperature of the air flow onto the human body.

## Patentansprüche

1. Kabine zum Unterbringen einer Person, wobei die Kabine ein Kabinenvolumen (4) definiert und Folgendes aufweist:
einen Basisabschnitt (6) und einen Oberabschnitt (8); Fließmittel, um Luft zu veranlassen, sich in einem Abwärtsluftstrom in der Kabine zu bewegen, wobei der Abwärtsluftstrom ein vorbestimmtes Volumen in der Kabine definiert;
Umwälzmittel (18) zum Umwälzen der Luft innerhalb der Kabine;
Filtermittel (20) zum Filtern der Luft innerhalb der Kabine; und
Sprühmittel (100; 30), um ein Produkt in mindestens einen Teil des Kabinenvolumens und auf einen Körper einer Person, die in der Kabine positioniert ist, zu sprühen, wobei das Fließmittel eine erste Sammelkammer (10) mit positivem Druck aufweist, die sich in dem Oberabschnitt der Kabine befindet, und eine zweite Sammelkammer (12) mit negativem Druck, die sich in der Basis der Kabine befindet, ein oder mehrere Lufteinlassgitter (104), die mit der ersten Sammelkammer verbunden sind, und ein oder mehrere Fußroste (102), die mit der zweiten Sammelkammer verbunden sind, wobei die erste und die zweite Sammelkammer miteinander über eine Rohrleitung (16) verbunden sind, **dadurch gekennzeichnet, dass** das Filtermittel ein erstes Filter (20a) zum Filtern nassen Feststoffmaterials aufweist, und ein zweites Filter (20d) zum Filtern trockenen Feststoffmaterials, wobei das erste Filter innerhalb der Rohrleitung in einem unteren Abschnitt der Rohrleitung, die zur zweiten Sammelkammer und zu dem einen oder den mehreren Fußrosten (102) gehört, positioniert ist, und das zweite Filter zu dem einen oder den mehreren Einlassgittern gehört.

2. Kabine (2; 28) nach Anspruch 1, die ferner Temperaturmittel (26) zum Steuern der Temperatur der innerhalb der Kabine zirkulierenden Luft aufweist.

3. Kabine (2; 28) nach Anspruch 2, wobei das Temperaturmittel den Luftstrom auf etwa 29 °C bis 30 °C halten kann.

4. Kabine (2) nach Anspruch 2, wobei das Temperaturmittel den Luftstrom auf etwa 33 °C halten kann.

5. Kabine (2) nach Anspruch 2, 3 oder 4, wobei das Temperaturmittel ein Heizgerät aufweist.

6. Kabine (2) nach einem der vorhergehenden Ansprüche, wobei die erste Sammelkammer (10) Luft mit Druck auf einen Druck über dem Luftdruck beaufschlagen kann, und wobei die zweite Sammelkammer (12) den Druck der Luft auf weniger als den Luftdruck senken kann.

7. Kabine (2) nach einem der vorhergehenden Ansprüche, wobei die Rohrleitung (16) mehrere Rohrleitungsabschnitte aufweist.

8. Kabine (2) nach einem der vorhergehenden Ansprüche, wobei das Umwälzmittel ein Gebläse aufweist.

9. Kabine (2) nach einem der vorhergehenden Ansprüche, wobei sich das Umwälzmittel innerhalb der Rohrleitung befindet.

10. Kabine (2) nach Anspruch 9, wobei das Umwälzmittel ein maximales Querschnittmaß hat und die Rohrleitung ein maximales Querschnittmaß hat, das im Wesentlichen größer ist als das Querschnittmaß des Umwälzmittels.

11. Kabine (2) nach einem der vorhergehenden Ansprüche, wobei das Filtermittel ein drittes Filter (20b) aufweist, das ein Filter mit niedriger Impedanz zum Filtern nassen Feststoffmaterials aufweist.

12. Kabine nach einem der vorhergehenden Ansprüche, wobei das Filtermittel ein viertes Filter (20c) aufweist, das ein zweites Filter mit niedriger Impedanz zum Filtern trockenen Feststoffmaterials aufweist.

13. Kabine nach einem der vorhergehenden Ansprüche, wobei das zweite Filter (20d) eine Impedanz hat, die über das Filter variiert.

14. Kabine nach einem der vorhergehenden Ansprüche, wobei das Filtermittel ein fünftes Filter (20e) aufweist, das eine erste Filterkomponente aufweist, die ein Filter mit relativ hoher Impedanz aufweist, und eine zweite Filterkomponente, die ein kombiniertes mechanisches und elektrostatisches Filter aufweist.

15. Kabine nach einem der vorhergehenden Ansprüche, wobei das vorbestimmte Volumen einen Abschnitt des Kabinenvolumens aufweist.

16. Kabine nach einem der vorhergehenden Ansprüche, wobei das Sprühmittel ein handgehaltenes Werkzeug (100) aufweist.

17. Kabine nach Anspruch 16, wobei das handgehaltene Werkzeug eine Luftpistole (100) aufweist.

18. Kabine nach Anspruch 17, wobei das handgehaltene Werkzeug einen druckluftlosen Zerstäuber aufweist.

19. Kabine nach einem der Ansprüche 1 bis 15, wobei das Sprühmittel ein dezentral bedienbares Werkzeug (30) aufweist.

20. Kabine nach Anspruch 19, wobei das dezentral bedienbare Werkzeug mehrere Düsen (34) aufweist, die das Produkt in das vorbestimmte Volumen sprühen können.

21. Kabine nach Anspruch 19, wobei das dezentral bedienbare Werkzeug ferner Einstellmittel (52, 53) zum Einstellen der Höhe der Düsen aufweist.

22. Kabine nach Anspruch 21, wobei das Einstellmittel zusätzlich die Haltung der Düsen einstellt.

23. Kabine nach einem der Ansprüche 19 bis 22, wobei das dezentral bedienbare Werkzeug (30) einen Düsenträger (54) aufweist, der im Wesentlichen eine Bogenform definiert, wobei die Düsen positioniert sind, um das Produkt in einem Bereich, der von dem Düsenträger definiert wird, zu sprühen.

24. Kabine nach einem der vorhergehenden Ansprüche, die ein dezentral bedienbares Werkzeug (30) aufweist, mit Mitteln zum automatischen Transportieren des Werkzeugs, um das Sprühen zwischen zwei Bereichen in der Kabine bereitzustellen.

25. Kabine nach Anspruch 24, wobei das automatische Transportmittel mindestens eine Läufereinheit (52, 53) aufweist, die vertikal zwischen zwei Positionen bewegt werden kann, wobei die Läufereinheit mindestens ein Sprühmittel trägt.

26. Kabine nach einem der vorhergehenden Ansprüche, die Sprühpistolen aufweist, die ausgerichtet sind, um Produkt horizontal und/oder mit einem Winkel zu der Horizontalen (entweder aufwärts oder abwärts) und/oder in irgendeiner Kombination dieser zu sprühen.

27. Verfahren zum Auftragen eines Produkts auf den menschlichen Körper, das die folgenden Schritte aufweist:
Veranlassen der Luft zum Fließen in eine Abwärtsrichtung auf den menschlichen Körper innerhalb einer Kabine (2), wobei die Kabine ein Kabinenvolumen (4) definiert und einen Basisabschnitt (6) und einen Oberabschnitt (8) aufweist;
Fließmittel, um die Luft zu veranlassen in einem Abwärtsluftstrom innerhalb der Kabine zu strömen, wobei der Abwärtsluftstrom ein vorbestimmtes Volumen innerhalb der Kabine definiert;
Umwälzmittel (18) zum Umwälzen der Luft innerhalb der Kabine;
Sprühmittel (20) zum Sprühen eines Produkts in mindestens einen Teil des Kabinenvolumens auf den menschlichen Körper, wobei das Fließmittel eine erste Sammelkammer (10) mit positivem Druck aufweist, die in dem Oberabschnitt der Kabine untergebracht ist, und eine zweite Sammelkammer (12) mit negativem Druck, die in der Basis der Kabine untergebracht ist, ein oder mehrere Einlassgitter (104), die mit der ersten Sammelkammer verbunden sind, und einen oder mehrere Fußroste (102), die zu der zweiten Sammelkammer gehören, wobei die erste und die zweite Sammelkammer miteinander über eine Rohrleitung (16) verbunden sind;
Umwälzen der Luft, die auf den menschlichen Körper strömt;
Filtern der Luft, um nasse und trockene Feststoffmaterialien zu entfernen;
Sprühen eines Produkts auf den menschlichen Körper, **dadurch gekennzeichnet, dass** der Schritt des Filterns der Luft zum Entfernen nasser und trockener Feststoffmaterialien das Durchgehen der Luft durch ein erstes Filter (20a) zum Filtern nassen Feststoffmaterials aufweist;
Durchgehen der Luft durch ein zweites Filter (20d) zum Filtern trockenen Feststoffmaterials, wobei
das erste Filter innerhalb der Rohrleitung in einem unteren Abschnitt der Rohrleitung positioniert ist, der zu der ersten Sammelkammer und dem einen oder mehreren Fußrosten (102) gehört, und das zweite Filter zu dem einen oder mehreren Einlassgittern gehört.

28. Verfahren nach Anspruch 27, das ferner einen Schritt des Durchgehens der Luft durch ein drittes Filter (20b) aufweist, das ein Filter mit niedriger Impedanz zum Filtern nassen Feststoffmaterials aufweist.

29. Verfahren nach Anspruch 27 oder 28, das ferner den Schritt des Durchgehens der Luft durch ein viertes Filter (20c) aufweist, das ein zweites Filter mit niedriger Impedanz zum Filtern trockenen Feststoffmaterials aufweist.

30. Verfahren nach einem der Ansprüche 27 bis 29, das den weiteren Schritt des Durchgehens der Luft durch ein fünftes Filter (20e) aufweist, das eine erste Filterkomponente aufweist, die ein Filter mit relativ hoher Impedanz aufweist, und eine zweite Filterkomponente, die ein kombiniertes mechanisches und elektrostatisches Filter aufweist.

31. Verfahren nach einem der Ansprüche 27 bis 30, das ferner den Schritt des Steuerns der Temperatur des Luftstroms auf den menschlichen Körper aufweist.

## Revendications

1. Cabine pour loger une personne, la cabine définissant un volume de cabine (4) et comprenant :
une portion de base (6) et une portion supérieure (8) ;
un moyen d'écoulement pour amener de l'air à se déplacer dans un écoulement d'air vers le bas au sein de la cabine, l'écoulement d'air vers le bas définissant un volume prédéterminé dans la cabine ;
un moyen de recirculation (18) pour faire recirculer l'air au sein de la cabine ;
un moyen de filtration (20) pour filtrer l'air au sein de la cabine ; et
un moyen de projection (100 ; 30) pour projeter un produit dans au moins une partie du volume de cabine et sur le corps d'une personne positionnée dans la cabine, dans laquelle le moyen d'écoulement comprend un premier plénum (10) de pression positive situé dans la portion supérieure de la cabine, et un second plénum (12) de pression négative situé dans la base de la cabine, une ou plusieurs grilles d'entrée d'air (104) associées au premier plénum et une ou plusieurs grilles pour pied (102) associées au second plénum, dans laquelle les premier et second plénums sont raccordés l'un à l'autre via un conduit (16), **caractérisée en ce que** le moyen de filtration comprend un premier filtre (20a) pour filtrer la matière particulaire humide ; et un deuxième filtre (20d) pour filtrer la matière particulaire sèche, où le premier filtre est positionné au sein du conduit dans une portion inférieure du conduit associé au second plénum et les une ou plusieurs grilles pour pied (102), et le deuxième filtre est associé à une ou plusieurs grilles d'entrée.

2. Cabine (2 ; 28) selon la revendication 1, comprenant en outre un moyen de température (26) pour réguler la température de l'air circulant au sein de la cabine.

3. Cabine (2 ; 28) selon la revendication 2, dans laquelle le moyen de température est adapté pour maintenir l'écoulement d'air à environ 29°C à 30°C.

4. Cabine (2) selon la revendication 2, dans laquelle le moyen de température est adapté pour maintenir l'écoulement d'air à environ 33 °C.

5. Cabine (2) selon les revendications 2, 3 ou 4, dans laquelle le moyen de température comprend un chauffage.

6. Cabine (2) selon l'une quelconque des revendications précédentes, dans laquelle le premier plénum (10) est adapté pour pressuriser l'air à une pression dépassant la pression ambiante, et le second plénum (12) est adapté pour dépressuriser l'air à une valeur inférieure à la pression atmosphérique.

7. Cabine (2) selon l'une quelconque des revendications précédentes, dans laquelle le conduit (16) comprend une pluralité de sections de conduit.

8. Cabine (2) selon l'une quelconque des revendications précédentes, dans laquelle le moyen de recirculation comprend un ventilateur.

9. Cabine (2) selon l'une quelconque des revendications précédentes, dans laquelle le moyen de recirculation est situé dans le conduit.

10. Cabine (2) selon la revendication 9, dans laquelle le moyen de recirculation a une dimension en coupe maximale, et le conduit a une dimension en coupe maximale qui est sensiblement plus grande que la dimension en coupe du moyen de recirculation.

11. Cabine (2) selon l'une quelconque des revendications précédentes, dans laquelle le moyen de filtration comprend un troisième filtre (20b) comprenant un filtre de faible impédance pour filtrer la matière particulaire humide.

12. Cabine selon l'une quelconque des revendications précédentes, dans laquelle le moyen de filtration comprend un quatrième filtre (20c) comprenant un deuxième filtre de faible impédance pour filtrer la matière particulaire sèche.

13. Cabine selon l'une quelconque des revendications précédentes, dans laquelle le deuxième filtre (20d) a une impédance qui varie à travers le filtre.

14. Cabine selon l'une quelconque des revendications précédentes, dans laquelle le moyen de filtration comprend un cinquième filtre (20e) comprenant un premier composant de filtre comprenant un filtre d'impédance relativement élevée, et un second composant de filtre comprenant un filtre mécanique et électrostatique combiné.

15. Cabine selon l'une quelconque des revendications précédentes, dans laquelle le volume prédéterminé comprend une portion du volume de cabine.

16. Cabine selon l'une quelconque des revendications précédentes, dans laquelle le moyen de projection comprend un outil à main (100).

17. Cabine selon la revendication 16, dans laquelle l'outil à main comprend un pistolet à air (100).

18. Cabine selon la revendication 17, dans laquelle l'outil à main comprend un pulvérisateur sans air.

19. Cabine selon l'une quelconque des revendications 1 à 15, dans laquelle le moyen de projection comprend un outil actionnable à distance (30).

20. Cabine selon la revendication 19, dans laquelle l'outil actionnable à distance comprend une pluralité de buses (34) adaptées pour projeter le produit dans le volume prédéterminé.

21. Cabine selon la revendication 19, dans laquelle l'outil actionnable à distance comprend en outre un moyen d'ajustement (52, 53) pour ajuster la hauteur des buses.

22. Cabine selon la revendication 21, dans laquelle le moyen d'ajustement ajuste en outre l'attitude des buses.

23. Cabine selon l'une quelconque des revendications 19 à 22, dans laquelle l'outil actionnable à distance (30) comprend un support de buse (54) définissant une forme sensiblement arquée, les buses étant positionnées pour pulvériser le produit dans une zone définie par le support de buse.

24. Cabine selon l'une quelconque des revendications précédentes, comprenant un outil actionnable à distance (30) avec un moyen pour transporter automatiquement l'outil pour assurer une pulvérisation entre deux zones dans la cabine.

25. Cabine selon la revendication 24, dans laquelle le moyen de transport automatique comprend au moins une unité de coulisse (52, 53) déplaçable verticalement entre deux positions, l'unité de coulisse supportant au moins un moyen de pulvérisation.

26. Cabine selon l'une quelconque des revendications précédentes, comprenant des pistolets de pulvérisation qui sont dirigés pour pulvériser un produit horizontalement et/ou selon un angle par rapport à l'horizontale (qu'il soit dirigé vers le haut ou vers le bas) et/ou l'une de leurs combinaisons.

27. Procédé pour appliquer un produit à un corps humain comprenant les étapes consistant à :
amener de l'air à s'écouler dans une direction vers le bas sur le corps humain au sein d'une cabine (2), la cabine définissant un volume de cabine (4) et comprenant une portion de base (6) et une portion supérieure (8) ;
un moyen d'écoulement pour amener l'air à s'écouler dans un écoulement d'air vers le bas au sein de la cabine, l'écoulement d'air vers le bas définissant un volume prédéterminé au sein de la cabine ;
un moyen de recirculation (18) pour faire recirculer l'air au sein de la cabine ;
et un moyen de projection (20) pour projeter un produit dans au moins une partie du volume de cabine sur le corps humain, le moyen d'écoulement comprenant un premier plénum (10) de pression positive situé dans la portion supérieure de la cabine, et un second plénum (12) de pression négative situé dans la base de la cabine, une ou plusieurs grilles d'entrée d'air (104) associées au premier plénum et une ou plusieurs grilles pour pied (102) associées au second plénum, les premier et second plénums étant raccordés l'un à l'autre via un conduit (16) ;
faire recirculer l'air circulant sur le corps humain ;
filtrer l'air pour éliminer la matière particulaire humide et sèche ;
projeter un produit sur le corps humain, **caractérisé en ce que** l'étape de filtration de l'air pour éliminer la matière particulaire humide et sèche comprend le fait de faire passer l'air sur un premier filtre (20a) pour filtrer la matière particulaire humide ;
faire passer l'air sur un deuxième filtre (20d) pour filtrer la matière particulaire sèche ; dans lequel
le premier filtre est positionné au sein du conduit dans une portion inférieure du conduit associé au second plénum et les une ou plusieurs grilles pour pied (102) et le deuxième filtre est associé à une ou plusieurs grilles d'entrée.

28. Procédé selon la revendication 27, comprenant l'étape supplémentaire consistant à faire passer l'air sur un troisième filtre (20b) comprenant un filtre de faible impédance pour filtrer la matière particulaire humide.

29. Procédé selon la revendication 27 ou la revendication 28, comprenant l'étape supplémentaire consistant à faire passer l'air sur un quatrième filtre (20c) comprenant un second filtre de faible impédance pour filtrer la matière particulaire sèche.

30. Procédé selon l'une quelconque des revendications 27 à 29, comprenant l'étape supplémentaire consistant à faire passer l'air sur un cinquième filtre (20e) comprenant un premier composant de filtre comprenant un filtre d'impédance relativement élevée, et un second composant de filtre comprenant un filtre mécanique et électrostatique combiné.

31. Procédé selon l'une quelconque des revendications 27 à 30, comprenant en outre une étape de régulation de la température de l'écoulement d'air sur le corps humain.
